# EUROPEAN PATENT SPECIFICATION

(11) **EP 4 110 236 B1**
(45) Date of publication and mention of the grant of the patent: **06.03.2024**
(21) Application number: 21704610.1
(22) Date of filing: 25.01.2021
(51) Int. Cl.: A61F 2/40, A61F 2/30

(54) **COUPLING SYSTEM OF THE COMPONENTS OF A SHOULDER JOINT PROSTHESIS**
VERBINDUNGSSYSTEM DER KOMPONENTEN EINER SCHULTERGELENKSPROTHESE
SYSTÈME DE CONNEXION DES COMPOSANTS D'UNE PROTHÈSE DE L'ARTICULATION DE L'ÉPAULE

(30) Priority: 27.02.2020 IT 202000000925 U
(43) Date of publication of application: 04.01.2023
(73) Proprietor: Permedica S.p.A., 23807 Merate (LC) (IT)
(72) Inventor: PEREGO, Marco, 23807 Merate (LC) (IT)
(74) Representative: Maccalli, Marco
(86) International application number: PCT/IB2021/050537
(87) International publication number: WO 2021/171108

(56) References cited:
- EP-A1- 1 656 910
- EP-A1- 3 598 957
- WO-A1-2011/098890
- WO-A1-2019/053576
- US-A1- 2004 220 673

## Description

The present invention is defined in claim 1 and relates to a coupling system between the parts in a prosthesis for the articulation of the shoulder.

Various prosthetic solutions for the articulation of the shoulder are known, which have couplings between the parts formed using components coupled in different ways.

The document WO 2019/053576 discloses a total shoulder prosthesis where it is possible to convert from an anatomical prosthesis to a reverse prosthesis replacing only the articular components without replacing the components implanted in the humeral and scapular bone.

A prosthesis of this type is also disclosed for example in EP1656910.

This specific solution provides that a first articulation element and a second articulation element be used for the articulation of a humerus in a scapula of a shoulder.

The first articulation element, for example, is at least partially concave, fixed to the humerus, and the second articulation element on the other hand is partially convex to be associated with the glenoid cavity. This second association is effected through an arrangement of metal fixing means.

As the second articulation element comprises an external part made of or coated in plastic or ceramic material, this solution proposes the provision of an annular element made of metal arranged rigidly blocked inside said external part. And said annular element is destined for receiving a pin fixing element for connection to a support which is inserted in the glenoid cavity.

As already mentioned, this is one of the possible forms of coupling which necessarily also involves a certain type of procedure for positioning the prosthesis.

The general objective of the present invention is to provide a coupling system or arrangement between the parts in a prosthesis for the articulation of the shoulder of a different type in order to be able to use different types of positioning procedures in parallel, suitable for particular specific requirements.

A further objective of the present invention is to provide a relatively stable and simple coupling system between the parts in a prosthesis for the articulation of the shoulder.

The above-mentioned objectives are achieved by a coupling system between the parts in a prosthesis for the articulation of the shoulder produced according to independent claim 1 and the following subordinate claims.

The structural and functional characteristics of the present invention and its advantages with respect to the known art will become even more evident from the following description, referring to the attached schematic drawings, which show an embodiment example of the same invention. In the drawings:
- figure 1 shows a raised side section of a first embodiment of a coupling system between the parts in a prosthesis for the articulation of the shoulder with component parts exploded together according to the present invention;
- figure 2 shows some of the parts shown in figure 1 in a perspective view which is also exploded;
- figure 3 shows a first coupling step for the coupling system shown in figure 1;
- figure 4 shows a second coupling step for the coupling system shown in figure 1;
- figure 5 shows a third coupling step for the coupling system shown in figure 1;
- figure 6 shows a fourth coupling step for the coupling system shown in figure 1;
- figure 7 shows the final arrangement of the coupling system shown in figure 1;
- figure 8 shows a raised side section of a second embodiment of a coupling system between the parts in a prosthesis for the articulation of the shoulder with component parts exploded together;
- figure 9 shows some of the parts shown in figure 8 in a perspective view also exploded;
- figure 10 shows a first coupling step for the coupling system shown in figure 8;
- figure 11 shows a second coupling step for the coupling system shown in figure 8;
- figure 12 shows a third coupling step for the coupling system shown in figure 8;
- figure 13 shows the final arrangement of the coupling system shown in figure 8.

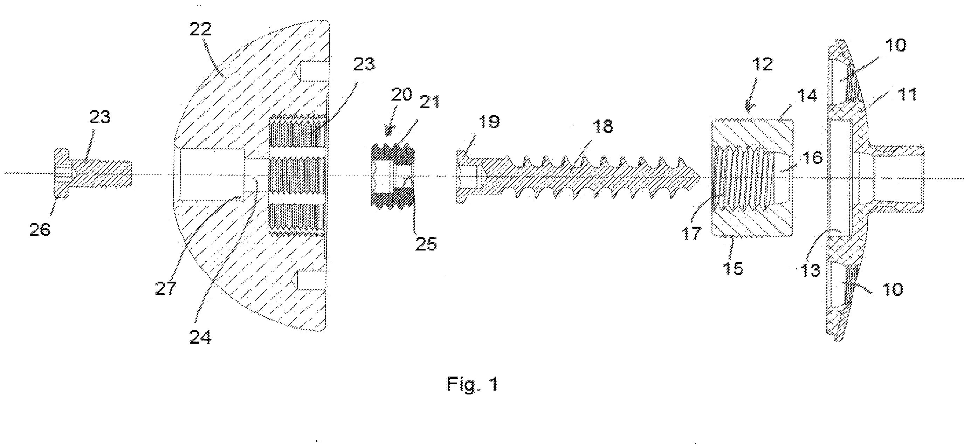

With reference to figures 1 to 7, exemplary and nonlimiting, a first embodiment of a coupling system is shown between the parts in a prosthesis for the articulation of the shoulder with component parts exploded together according to the present invention.

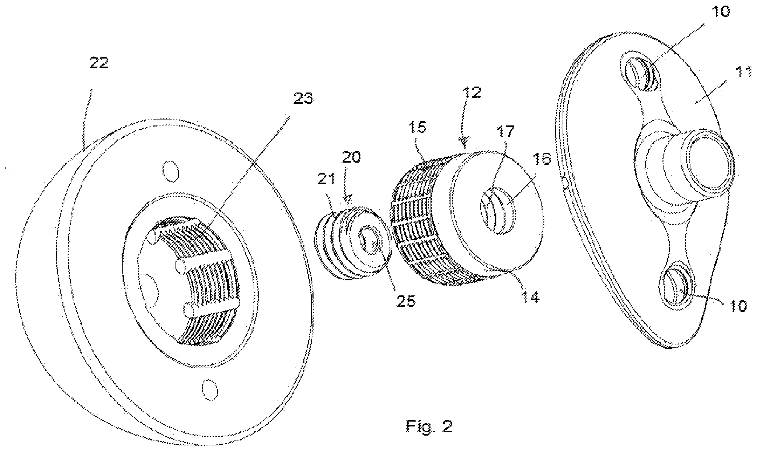

The coupling system in the parts illustrated of a prosthesis for the articulation of the shoulder shown in figures 1 and 2 comprises a metal support 11 which is suitable for being pressure-inserted in a seat formed in the glenohumeral bone and fixed therein with screws (not shown) arranged in holes 10. The metal support 11 has been previously specifically prepared and milled.

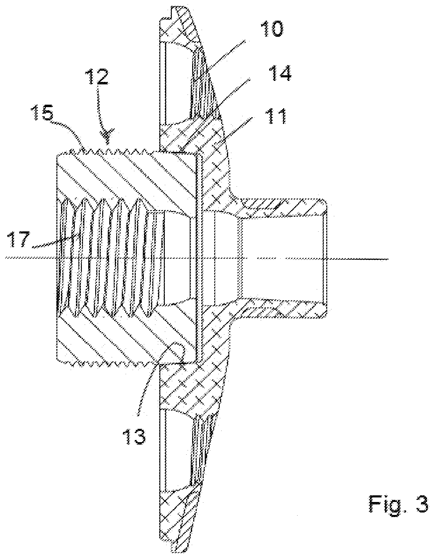

At this point, as shown in figure 3, a pin element 12 is arranged in a hole 13 of the metal support 11. It should be noted that this coupling is effected thanks to the fact that the pin element 12 provides a conical section 14 at one end, which engages with a complementary internal conical shape of the hole 13 by means of a morse cone. The pin element 12 at its other end provides a cylindrical section 15 with an external knurled surface. Furthermore, this pin element 12 has a hole which passes through a section 16 corresponding to the conical section 14 and is threaded in a section 17 corresponding to the cylindrical section 15.

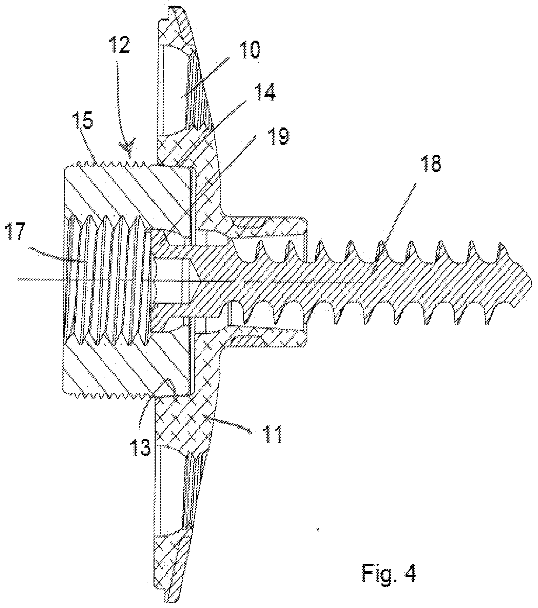

Once this coupling step has been carried out, a screw 18 is inserted in the hole 17/16 (figure 4) so that the end of the screw 18 is screwed into a hole prepared with a special cutter in the scapular bone.

And continuing the screwing of the screw 18, one of its heads 19 becomes abutted in the section 16 of the hole of the pin element 12 and further stabilizes in compression the pin element 12 against the metal support 11.

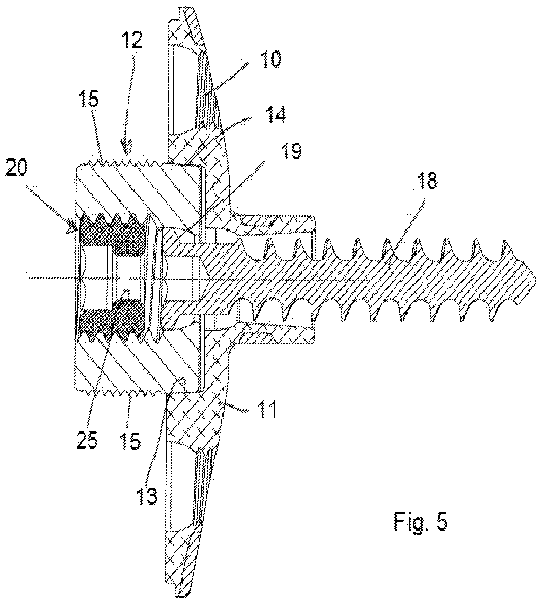

This is followed by a further step for the insertion of a cylindrical bush 20 externally threaded in 21 inside the threaded section 17 of the pin element 12 with screwing; and this after the pin element 12, as specified and shown, has been made almost integral with the metal support 11 (figure 5).

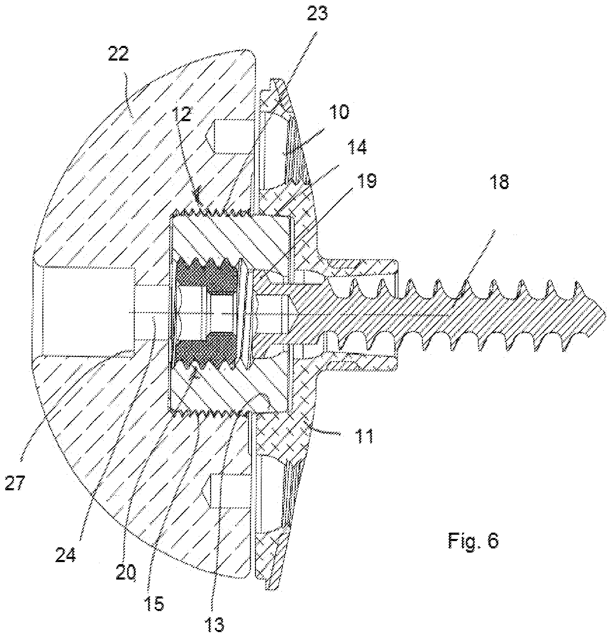

At this point, with the subgroup indicated above thus stably formed, the final positioning of a glenosphere 22, or hemispherical element, is effected by compression in axis guided with a specific tool or screwing mechanism in a guided and controlled manner, until the final arrangement has been reached, as shown in figure 6. In particular, said glenosphere 22 provides a pass-through axial hole 24 with sections having a differentiated diameter. One side facing the elements previously described, in fact, provides an internal seat with an enlarged diameter 23' provided with knurling which is suitable for being inserted above and coupled with the cylindrical section 15 with an external knurled surface of the pin element 12. This causes the stable blocking of the assembly formed integral with the metal support 11, which in turn had been previously pressure-inserted in the seat formed in the glenohumeral bone.

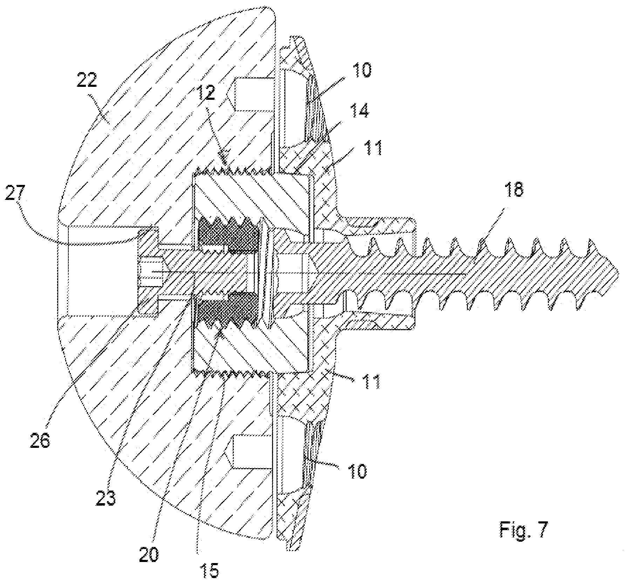

All that remains therefore is simply a last step in which a safety screw 23 is screwed in, which, passing through the axial pass-through hole 24 of the glenosphere 22, is positioned in an internal threaded portion 25 of the cylindrical bush 20 (figure 7) which had previously been firmly screwed into the pin element 12. It should also be noted how a head 26 of the safety screw 23 is stably arranged in a section with an enlarged diameter 27 of the axial hole 24, arranged on the opposite side of the glenosphere 22 with respect to that of the internal seat having an enlarged diameter 23' provided with knurling.

The system according to the invention essentially comprises a metal support 11, a pin element 12, a cylindrical bush 20 and a glenosphere or hemispherical element 22 which are consecutively coupled in the sequence listed above, in addition to reciprocal constraint elements.

This arrangement shown in figure 7 therefore shows a first embodiment of a coupling system between the parts in a prosthesis for the articulation of the shoulder according to the present invention and how it is assembled.

This arrangement is particularly simple and easy to assemble, and its parts are also very simple to construct.

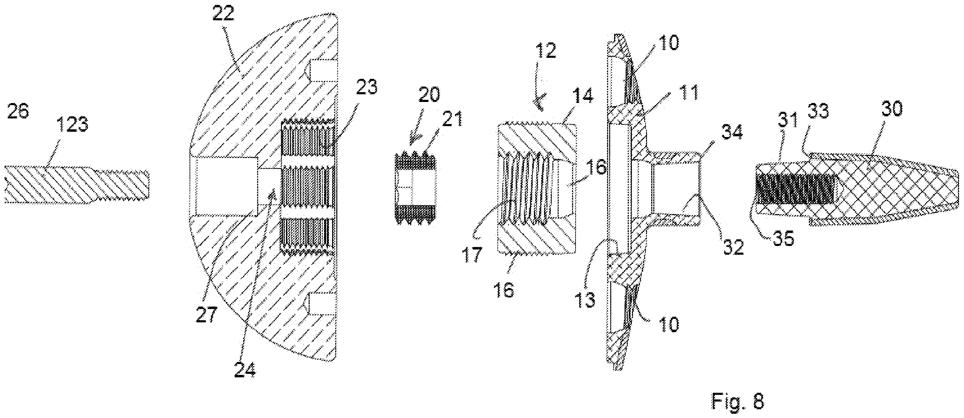

Figures 8 to 13 show a second embodiment of a coupling system between the parts in a prosthesis for the articulation of the shoulder according to the present invention.

In this second embodiment, the same reference numbers will be used for identical elements.

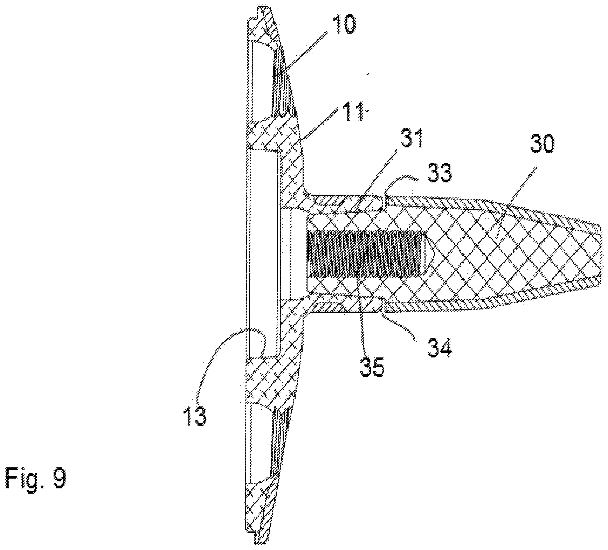

In figure 8 which shows all of the components of the system forming part of this embodiment, it can be seen that in addition to the metal support 11 there is also a central peg 30 which in a first step (figure 9) is coupled with the support 11. An end having a reduced diameter 31 of the peg 30 is in fact inserted into a pass-through hole 32 formed at the end of the support 11.

More specifically, this conical end 31 is inserted into and engages in the pass-through hole 32 formed in said metal support 11 having a complementary internal conical shape by means of a morse cone.

The peg 30, in its interior, also provides a threaded hole 35 at the end facing the metal support 11. The whole thus composed (figure 9) is pressure-inserted into a seat formed in the glenohumeral bone and possibly further fixed therein with screws to the scapular bone (not shown) passing through the holes 10 of the support 11.

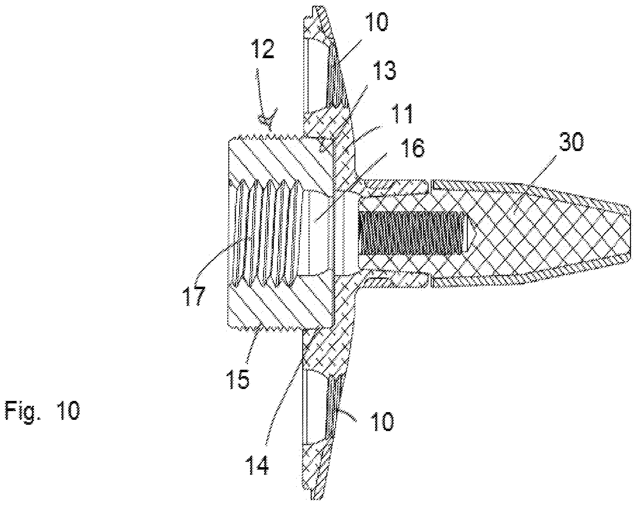

At this point, what has already been indicated for the embodiment of figure 3 is effected, as the same pin element 12 is provided, which is arranged in the hole 13 of the metal support 11. The pin element 12 through its end with the conical section 14 is therefore engaged in the complementary internal conical shape of the hole 13 (figure 10). And also here, it is evident that the pin element 12 at its other end provides the cylindrical section 15 with an external knurled surface. The pin element 12 provides a pass-through hole with the section 16 corresponding to the conical section 14 and is threaded in the section 17 corresponding to the cylindrical section 15.

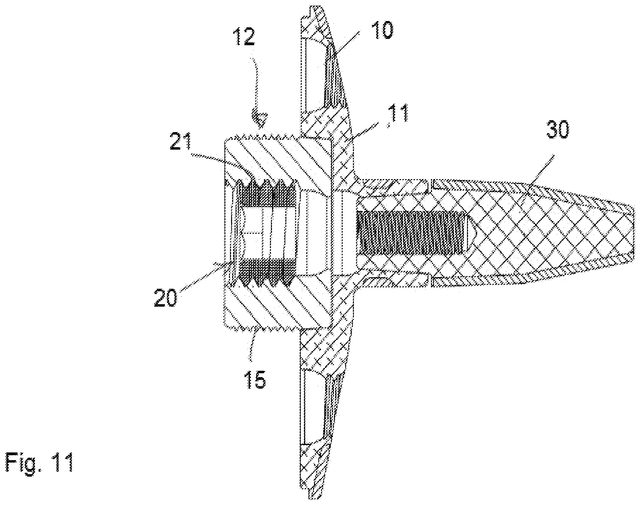

Once this coupling step has been carried out, unlike what is provided for in the first embodiment, the step for inserting the cylindrical bush 20 externally threaded in 21 is effected within the threaded section 17 of the pin element 12 with screwing (figure 11). This is effected after the pin element 12, as already indicated and shown, has been made almost integral with the metal support 11.

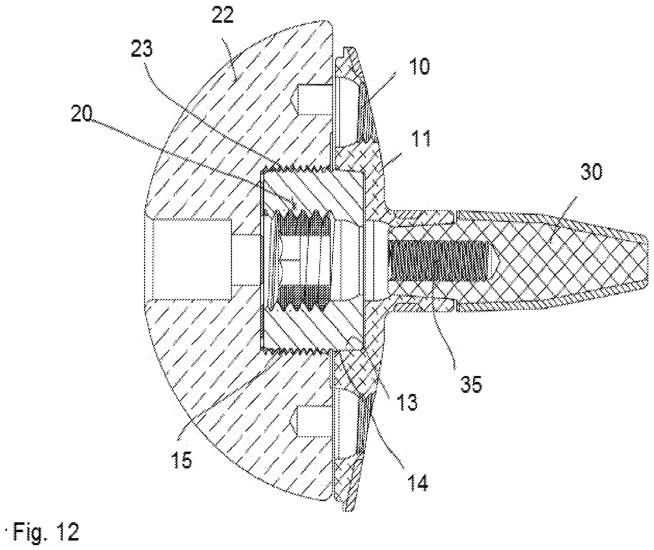

This is followed by a step in which the final positioning of the glenosphere 22 is effected on the subgroup indicated above, thus stably formed, by means of compression in axis guided with a specific tool or screwing mechanism in a guided and controlled manner, until the final arrangement has been reached, as shown in figure 12. Also in this case, in fact, the glenosphere 22 provides that the internal seat 23' be provided with knurling which is inserted above the cylindrical section 15 with an external knurled surface of the pin element 12. This causes the stable blocking of the assembly formed integral with the metal support 11, which in turn had been previously pressure-inserted into the seat formed in the glenohumeral bone.

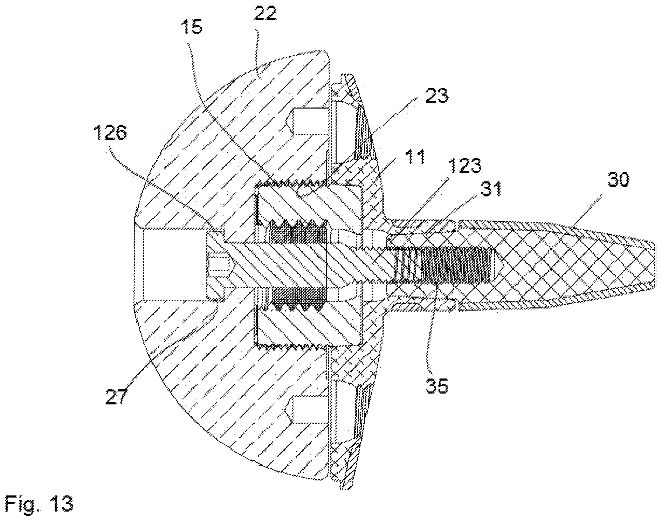

All that remains therefore is simply a last step (shown in figure 13) in which a safety screw 123 is screwed in, which is longer than the previous safety screw 23. Said screw 123 passes through the axial pass-through hole 24 of the glenosphere 22 and then passes inside the cylindrical bush 20, inside the pin element 12. It then continues, passing through a first section of the metal support 11 and is positioned in the threaded hole 35 of the peg 30, previously arranged in the metal support 11. Here again, a head 126 of the safety screw 123 is stably arranged in a section with an enlarged diameter 27 of the axial hole 24.

This screw 123 is therefore inserted through the hole of the bush 20 and is screwed stably to the threaded hole 35 of the peg 30.

This arrangement illustrated in figure 13 therefore shows a second embodiment of the coupling system between the parts in a prosthesis for the articulation of the shoulder according to the present invention and how it is assembled.

It should be added that all the components described above are made of titanium alloy or, for example, the support and the peg of titanium alloy powder. An exception is the glenosphere 22 or hemispherical element which is instead preferably made entirely of UHMWPE (ISO5834/1/2) or UHMWPE containing vitamin E (ASTM F2695-12).

The objective mentioned in the preamble of the description has thus been achieved.

The protection scope of the present invention is defined by the enclosed claims.

## Claims

1. A coupling system between the parts in a prosthesis for the articulation of the shoulder comprising a metal support (11), a pin element (12), a cylindrical bush (20) and a glenosphere or hemispherical element (22) which are consecutively coupled in the sequence listed above, in addition to reciprocal constraint elements, wherein said metal support (11) is provided with a hole (13),
said metal support (11) and said pin element (12) are constrained with an interposed conical coupling (13,14), the pin element (12) being provided, externally at one of its ends, with a conical section (14), which engages with a complementary internal conical shape of the hole (13) by means of a morse cone,
wherein said pin element (12) provides a central pass-through hole which comprises a threaded section (17),
wherein said cylindrical bush (20) is externally provided with a threading (21) complementary to the threaded section (17) of the pin element (12),
wherein said glenosphere or hemispherical element (22) comprises an axial pass-through hole (24) having a first diameter,
and further having on one side an internal seat (23') with a second enlarged diameter provided with knurling,
wherein said reciprocal constraint elements comprise at least one safety screw (23, 123) having a head (26, 126),
wherein (i) said at least one safety screw (23) is adapted to be screwed into an internal threaded portion (25) of the cylindrical bush (20),
or wherein (ii) the coupling system further comprises a central peg (30) adapted to be coupled to the support (11), wherein said at least one safety screw (123) is adapted to be screwed into a threaded hole (35) in the interior of the central peg (30),
wherein said pin element (12) provides externally, at one end opposite to that of coupling with the metal support (11), a cylindrical section (15) with a knurled surface, said knurling of the internal seat (23') with the second enlarged diameter of the axial pass-through hole (24) of the glenosphere or hemispherical element (22) is complementary to the section (15) with a knurled surface of the pin element (12),
**characterized in that**
the axial pass-through hole (24) has a section with a third enlarged diameter (27) on the opposite side of the internal seat (23') having the second enlarged diameter, wherein the head (26, 126) of the at least one safety screw (23, 123) is adapted to be arranged in said section with the third enlarged diameter (27) of the axial hole (24) of the glenosphere.

2. The coupling system according to claim 1 option (i), further comprising a further screw (18) which passes through the pin element (12) and blocks it against the metal support (11).

3. The coupling system according to claim 1 option (ii), wherein said peg (30) has an end with a reduced diameter (31) which is inserted into a pass-through hole (32) formed in said metal support (11).

4. The coupling system according to one or more of the previous claims, wherein all of said elements are made of titanium alloy with the exception of said glenosphere (22) which is made entirely of UHMWPE in accordance with IS05834/1/2 or UHMWPE containing vitamin E in accordance with ASTM F2695-12.

## Patentansprüche

1. Ein Verbindungssystem zwischen den Teilen in einer Prothese für die Artikulation der Schulter, aufweisend einen Metallträger (11), ein Stiftelement (12), eine zylindrische Hülse (20) und eine Glenosphäre oder ein halbkugelförmiges Element (22), die nacheinander in der oben angegebenen Reihenfolge verbunden sind, zusätzlich zu wechselseitigen Hemmungselementen, wobei der Metallträger (11) mit einem Loch (13) versehen ist,
wobei der Metallträger (11) und das Stiftelement (12) mit einer dazwischenliegenden konischen Verbindung (13, 14) gehemmt sind, wobei das Stiftelement (12) außen an einem seiner Enden mit einem konischen Querabschnitt (14) versehen ist, der in eine komplementäre innere konische Form des Lochs (13) mittels eines Morsekegels eingreift,
wobei das Stiftelement (12) eine zentrales Durchgangsloch vorsieht, das einen Gewindeabschnitt (17) aufweist,
wobei die zylindrische Hülse (20) außen mit einem zum Gewindeabschnitt (17) des Stiftelements (12) komplementären Gewinde (21) versehen ist,
wobei die Glenosphäre oder das halbkugelförmige Element (22) ein axiales Durchgangsloch (24) mit einem ersten Durchmesser aufweist,
und ferner auf einer Seite einen Innensitz (23') mit einem zweiten vergrößerten Durchmesser aufweist, der mit einer Rändelung versehen ist,
wobei die wechselseitigen Hemmungselemente mindestens eine Sicherheitsschraube (23, 123) mit einem Kopf (26, 126) aufweisen,
wobei (i) die mindestens eine Sicherheitsschraube (23) so angepasst ist, dass sie in einen Innengewindeabschnitt (25) der zylindrischen Hülse (20) geschraubt werden kann,
oder wobei (ii) das Verbindungssystem außerdem einen zentralen Zapfen (30) aufweist, der angepasst ist, um mit dem Träger (11) verbunden zu werden, wobei die mindestens eine Sicherheitsschraube (123) so angepasst ist, dass sie in ein Gewindeloch (35) im Inneren des zentralen Zapfens (30) geschraubt werden kann,
wobei das Stiftelement (12) außen an einem Ende, das der Verbindung mit dem Metallträger (11) gegenüberliegt, einen zylindrischen Abschnitt (15) mit einer gerändelten Oberfläche aufweist,
wobei die Rändelung des Innensitzes (23') mit dem zweiten vergrößerten Durchmesser des axialen Durchgangslochs (24) der Glenosphäre oder des halbkugelförmigen Elements (22) komplementär zu dem Abschnitt (15) mit einer gerändelten Oberfläche des Stiftelements (12) ist,
**dadurch gekennzeichnet, dass**
das axiale Durchgangsloch (24) einen Abschnitt hat mit einem dritten vergrößerten Durchmesser (27) auf der gegenüberliegenden Seite des Innensitzes (23'), der den zweiten vergrößerten Durchmesser aufweist,
wobei der Kopf (26, 126) der mindestens einen Sicherheitsschraube (23, 123) so angepasst ist, um in dem Abschnitt mit dem dritten vergrößerten Durchmesser (27) des axialen Durchgangslochs (24) der Glenosphäre angeordnet zu sein.

2. Das Verbindungssystem nach Anspruch 1, Option (i),
das ferner eine weitere Schraube (18) aufweist, die
durch das Stiftelement (12) hindurchführt und es gegen den Metallträger (11) sperrt.

3. Das Verbindungssystem nach Anspruch 1, Option (ii),
wobei der Zapfen (30) ein Ende mit einem reduzierten Durchmesser (31) hat, das in ein im Metallträger (11) ausgebildetes Durchgangsloch (32) eingeführt ist.

4. Das Verbindungssystem nach einem oder mehreren der vorhergehenden Ansprüche, bei dem alle diese Elemente aus einer Titanlegierung hergestellt sind, mit Ausnahme der Glenosphäre (22), die vollständig aus UHMWPE gemäß ISO5834/l/2 oder UHMWPE, das Vitamin E enthält, gemäß ASTM F2695-12 gefertigt ist.

## Revendications

1. Système de couplage entre les parties d'une prothèse pour l'articulation de l'épaule comprenant un support métallique (11), un élément de broche (12), une douille cylindrique (20) et une glénosphère ou un élément hémisphérique (22) qui sont couplés successivement dans l'ordre indiqué précédemment, en plus d'éléments de contrainte réciproques, dans lequel ledit support métallique (11) est muni d'un orifice (13) ;
ledit support métallique (11) et ledit élément de broche (12) sont contraints avec un accouplement conique interposé (13, 14), l'élément de broche (12) étant muni, à l'extérieur d'une de ses extrémités, d'une section conique (14) qui s'engage avec une forme complémentaire conique intérieure de l'orifice (13) au moyen d'un cône Morse,
dans lequel ledit élément de broche (12) fournit un orifice traversant central qui comprend une section filetée (17),
dans lequel ladite douille cylindrique (20) est munie à l'extérieur d'un filetage (21) complémentaire à la section filetée (17) de l'élément de broche (12),
dans lequel ladite glénosphère ou ledit élément hémisphérique (22) comprend un orifice traversant axial (24) ayant un premier diamètre et ayant en outre sur un côté un siège interne (23') ayant un deuxième diamètre élargi muni d'un moletage,
dans lequel lesdits éléments de contrainte réciproques comprennent au moins une vis de sécurité (23, 123) ayant une tête (26, 126),
dans lequel (i) ladite au moins une vis de sécurité (23) est adaptée pour être vissée dans une partie filetée interne (25) de la douille cylindrique (20), ou
dans lequel (ii) le système de couplage comprend en outre une cheville centrale (30) adaptée pour être couplée au support (11), dans lequel ladite au moins une vis de sécurité (123) est adaptée pour être vissée dans un orifice fileté (35) à l'intérieur de la cheville centrale (30),
dans lequel ledit élément de broche (12) fournit extérieurement, au niveau d'une extrémité opposée à celle d'accouplement avec le support métallique (11), une section cylindrique (15) ayant une surface moletée, ledit moletage du siège interne (23') ayant le deuxième diamètre élargi de l'orifice traversant axial (24) de la glénosphère ou de l'élément hémisphérique (22) est complémentaire à la section (15) ayant une surface moletée de l'élément de broche (12),
**caractérisé en ce que**
l'orifice traversant axial (24) a une section ayant un troisième diamètre élargi (27) du côté opposé du siège interne (23') ayant le deuxième diamètre élargi,
dans lequel la tête (26, 126) de ladite au moins une vis de sécurité (23, 123) est adaptée pour être disposée dans ladite section ayant le troisième diamètre élargi (27) de l'orifice axial (24) de la glénosphère.

2. Système de couplage selon la revendication 1 option (i), comprenant en outre une vis supplémentaire (18) qui traverse l'élément de broche (12) et le bloque contre le support métallique (11) .

3. Système de couplage selon la revendication 1 option (ii), dans lequel ladite cheville (30) a une extrémité ayant un diamètre réduit (31) qui est insérée dans un orifice traversant (32) formé dans ledit support métallique (11).

4. Système de couplage selon l'une quelconque des revendications précédentes, dans lequel tous lesdits éléments sont constitués d'un alliage de titane à l'exception de ladite glénosphère (22) qui est entièrement constituée d'UHMWPE conforme à ISO5834/1/2 ou d'UHMWPE contenant de la vitamine E conforme à ASTM F2695-12.
